(19) Europäisches Patentamt
European Patent Office
Office européen des brevets

(11) **EP 2 305 355 A1**

(12) **EUROPEAN PATENT APPLICATION**
published in accordance with Art. 153(4) EPC

(43) Date of publication:
06.04.2011 Bulletin 2011/14

(51) Int Cl.:
*A63B 23/00* (2006.01)

(21) Application number: 09770143.7

(22) Date of filing: 23.06.2009

(86) International application number:
PCT/JP2009/061389

(87) International publication number:
WO 2009/157433 (30.12.2009 Gazette 2009/53)

(84) Designated Contracting States:
AT BE BG CH CY CZ DE DK EE ES FI FR GB GR
HR HU IE IS IT LI LT LU LV MC MK MT NL NO PL
PT RO SE SI SK TR
Designated Extension States:
AL BA RS

(30) Priority: 24.06.2008 JP 2008164992

(71) Applicant: Panasonic Electric Works Co., Ltd.
Kadoma-shi
Osaka 571-8686 (JP)

(72) Inventors:
• OCHI, Kazuhiro
Kadoma-shi
Osaka 571-8686 (JP)

• SHINOMIYA, Youichi
Kadoma-shi
Osaka 571-8686 (JP)
• GOTOU, Takao
Kadoma-shi
Osaka 571-8686 (JP)
• OZAWA, Takahisa
Kadoma-shi
Osaka 571-8686 (JP)

(74) Representative: Appelt, Christian W.
Forrester & Boehmert
Pettenkoferstrasse 20-22
80336 München (DE)

(54) **METHOD AND SYSTEM FOR CARRYING OUT SIMULATION AND MEASUREMENT RELATING TO OPTIMUM OPERATIONAL CONDITION FOR SUPPORT STAND OF PASSIVE TRAINING DEVICE**

(57) A simulator obtains muscle activities and joint contact forces by a computer simulation according to an operating condition for moving a support base of a passive exercise machine. A condition limiting unit finds intermediate conditions corresponding to desirable muscle activities and joint contact forces from muscle activities and joint contact forces obtained with the simulator according to different operating conditions. A motion simulator moves the support base according to the intermediate conditions. A myoelectric measurement device measures myoelectric potential of a subject supported by the support base. An evaluation device selects an operating condition corresponding to a larger muscle activity quantity from measurement results of muscle activity to define it as an operating condition of the passive exercise machine.

*FIG. 1*

EP 2 305 355 A1

## Description

TECHNICAL FIELD

The invention relates to method and system of simulation and measurement which can decide an optimum operating condition for a support base of a passive exercise machine, for example, an optimum operating condition for applying relatively heavy muscle load and light joint load to a user through the support base.

BACKGROUND ART

There are various proposed passive exercise machines configured to move a support base for supporting a part of user's body to change the user's posture.
For example, Japanese Patent Application Publication Number 2004-344684 published on December 9, 2004 discloses a balance exercise machine. This machine is configured to simulate horse riding exercise by oscillating a support base on which a user can ride to sit.
Japanese Patent Application Publication Number 2006-122595 published on May 18, 2006 discloses a swing exercise machine. This machine has a support base that can support the buttocks or the waist of a user in a posture for supporting a part of the user's own weight by feet (a sitting posture). The machine is configured to change a ratio of the user's own weight acting on the user's legs by oscillating the support base. There is also a machine having a support base on which a user can rest the user's feet in the standing position. This machine is configured to provide a user with walking motion or exercise for spreading movable ranges of joints of legs by moving the support base to change positions or orientations of the user's feet.
In a passive exercise machine, if the operating condition of the support base is changed, load amplitude acting on each muscle or load amplitude acting on each joint is changed. That is, the operating condition of the support base is changed and thereby muscle type on which a load mainly acts or activity degree of the muscle is changed and a joint contact force (joint force) of each joint is also changed. Preferably, the operating condition of the support base is decided so that a joint contact force is reduced and a muscle activity is enhanced.
A passive exercise machine is actually used by subjects at the present time in order to obtain a relationship an operating condition of the support base as well as a muscle activity and a joint contact force. In this case, the operating condition of the support base is changed and thereby change (process) of a muscle activity or a joint contact force is measured. A muscle activity can be obtained by measuring myoelectric potential of a subject. However, a joint contact force cannot be measured directly and accordingly the technology for estimating a joint contact force has been adopted (see e.g., Japanese Patent Application Publication Number 2006-034640 published on February 9, 2006). This is, a force acting on a measurable region is measured and a computer simulation is performed by using a model simulating a human body. In this technology, a shear force acting on a joint is obtained as a joint contact force.
If a muscle activity or a joint contact force is found by actually activating a passive exercise machine, the number of actual measurable operating conditions a day is about 20 at the most. Therefore, in order to obtain an optimum operating condition for enhancing the activity of a target muscle(s) and reducing the joint contact force, huge amounts of time are required. In addition, there is a problem that a heavy burden is imposed on a subject.
Also, a large joint contact force may generate by an operating condition of the support base. Therefore, in order to perform actual measurement in consideration of subject's safety, it is necessary to perform measurement while removing operating conditions by which a large joint contact force is expected to be generated. As result, a selection range of operating conditions may be narrowed beyond necessity and a below optimum operating condition may be selected.
Moreover, all motions of a person's actual muscle by using a passive exercise machine cannot be obtained by a computer simulation. On the other hand, an amount of muscle discharge (electrical activity of muscle) may be small in an extensor reflex. Accordingly, deciding a muscle used amount only by an amount of muscle discharge which can be observed from an electromyogram may make a wrong decision.

DISCLOSURE OF THE INVENTION

It is an object of the present invention: to evaluate a muscle activity and a joint contact force with respect to many operating conditions for a comparatively short time by using a computer simulation; to make it possible to decide an optimum operating condition, by also evaluating a subject's myoelectric potential, through a combination of the computer simulation and measurement of an amount of muscle discharge instead of deciding an operating condition of a real machine only by the computer simulation; and to optimize an operating condition provided for a real machine.
The present invention is a method of simulation and measurement related to an optimum operating condition for a support base of a passive exercise machine. The passive exercise machine comprises the support base configured to support all or part of body weight of a user, and a drive unit configured to move the support base. The passive exercise machine is configured to provide passive exercise for the user by moving the support base through the drive unit in accordance with an operating condition. The invention comprises computer implemented steps of: (a) obtaining different muscle activities and different joint contact forces in a target region of the user by a computer simulation sequentially according to each of operating conditions, the different muscle activities being activities of different muscles, the different joint contact forces being joint contact forces on different joints; (b) obtaining intermediate conditions from the operating conditions, the intermediate conditions being obtained by, if every muscle activity and every joint contact force obtained sequentially according to each of the operating conditions are in predetermined muscle activity and joint contact force ranges, respectively, including the operating condition among the intermediate conditions; (c) measuring myoelectric potential of a subject on the support base while controlling a motion simulator configured to move the support base in degrees-of-freedom sequentially according to each of the intermediate conditions; and (d) deciding and outputting an optimum operating condition from the intermediate conditions based on each myoelectric potential measured sequentially according to each of the intermediate conditions.

In this invention, muscle activities and joint contact forces are estimated with regard to different operating conditions by a computer simulation without using an actual passive exercise machine. Accordingly, muscle activities and joint contact forces can be evaluated with regard to many operating conditions for a comparatively short time. However, muscle activities and joint contact forces obtained by the computer simulation have a margin of error each. Especially, in case many operating conditions are evaluated for a short time, it is necessary to use a simple model having few parameters as a model for the simulation. Therefore, if an operating condition is decided only by a computer simulation with regard to muscle activities and joint contact forces, there is a possibility that an optimum operating condition cannot be selected.

Therefore, the invention obtains intermediate conditions by selecting, from computer simulation results, operating conditions that different muscle activities and different joint contact forces in a target region of the user are in objective ranges. An optimum operating condition is obtained from the intermediate conditions. That is, the support base's motion that meets an intermediate condition obtained by a computer simulation is realized by controlling, by each intermediate condition, the motion simulator for moving the support base in degrees-of-freedom. Myoelectric potential of a subject on the support base is measured, and the operating condition of the support base is decided from myoelectric potential measurement results per intermediate condition.

Thus, since the subject's myoelectric potential is actually measured by using intermediate conditions narrowed down by a computer simulation, various operating conditions can be generally evaluate for a short time. When evaluated operating conditions are narrowed down to intermediate conditions, it is possible to refine so that muscle-strengthening effect is emphasized or subject's safety is emphasized. Evaluation is performed based on an actual measurement of myoelectric potential with respect to the refined intermediate conditions, and accordingly time for the actual measurement of myoelectric potential is reduced, and almost optimum condition can be decided for a comparatively short time.

In an embodiment, the step (a) comprising: estimating position change over time of a human body joint of the user from position change over time of at least one inverted pendulum when the inverted pendulum is forcibly oscillated sequentially according to each of the operating conditions, the inverted pendulum being a human body model; and obtaining the different muscle activities and the different joint contact forces by applying the estimated position change to a musculo-skeletal model. In this embodiment, position change over time of a joint is estimated by relating the motion of the passive exercise machine to an inverted pendulum model that is a machine vibration model. A muscle activity and a joint contact force are estimated by using the musculo-skeletal model and applying position change of a joint to the musculo-skeletal model. Accordingly, a muscle activity and a joint contact force can be obtained by a comparatively small operation amount. That is, a computer simulation can be performed for a comparatively short time with regard to many operating conditions.

In an embodiment, the step (d) comprises: obtaining a maximum average value or a maximum peak value of muscle discharge from each myoelectric potential measured sequentially according to each of the intermediate conditions; and defining the intermediate condition corresponding to the maximum average value or the maximum peak value as the optimum operating condition. In this embodiment, an operating condition having a maximum average value or a maximum peak value for different amounts of muscle discharge is decided as an operating condition of the passive exercise machine. Accordingly, it is possible to automatically decide an optimum operating condition from the intermediate conditions based on subject's myoelectric potential.

In an embodiment, the joint contact force range is equal to or less than a specified value. The muscle activity range is a range from a first muscle activity to a second muscle activity. The first muscle activity is the largest muscle activity of each muscle activity obtained according to the operating conditions which are in the joint contact force range. The second muscle activity is a muscle activity lower than the first muscle activity by a defined number, of each muscle activity obtained according to the operating conditions which are in the joint contact force range. In this embodiment, intermediate conditions can be automatically selected.

The present invention is a system of simulation and measurement related to an optimum operating condition for a support base of a passive exercise machine. The passive exercise machine comprising the support base configured to support all or part of body weight of a user and a drive unit configured to move the support base. The passive exercise machine is configured to provide passive exercise for the user by moving the support base through the drive unit in accordance with an operating condition. The invention comprises a simulator, a motion simulator, a myoelectric measurement device, and an evaluation device. The simulator is configured (i) to obtain different muscle activities and different joint contact forces in a target region of the user by a computer simulation sequentially according to each of operating conditions, and (ii) to obtain intermediate conditions from the operating conditions. The different muscle activities are activities of different muscles. The different joint contact forces are joint contact forces on different joints. The intermediate conditions are obtained by, if every muscle activity and every joint contact force obtained sequentially according to each of the operating conditions are in predetermined muscle activity and joint contact force ranges, respectively, including the operating condition among the intermediate conditions. The motion simulator is configured to move the support base in degrees-of-freedom sequentially according to each of the intermediate conditions. The myoelectric measurement device is configured to measure myoelectric potential of a subject on the support base. The evaluation device is configured to decide and output an optimum operating condition from the intermediate conditions based on each myoelectric potential measured sequentially according to each of the intermediate conditions. The invention has the same advantage as the corresponding method.

In an embodiment, the simulator comprises a balance simulator and a musculoskeletal simulator. The balance simulator is configured to estimate position change over time of a human body joint of the user from position change over time of at least one inverted pendulum when the inverted pendulum is forcibly oscillated sequentially according to each of the operating conditions, the inverted pendulum being a human body model. The musculoskeletal simulator is configured to obtain the different muscle activities and the different joint contact forces by applying the estimated position change to a musculoskeletal model. The invention has the same advantage as the corresponding method.

In an embodiment, the evaluation device is configured: to obtain a maximum average value or a maximum peak value of muscle discharge from each myoelectric potential measured sequentially according to each of the intermediate conditions; and to define the intermediate condition corresponding to the maximum average value or the maximum peak value as the optimum operating condition. The invention has the same advantage as the corresponding method.

In an embodiment, the joint contact force range is equal to or less than a specified value. The muscle activity range is a range from a first muscle activity to a second muscle activity. The first muscle activity is the largest muscle activity of each muscle activity obtained according to the operating conditions which are in the joint contact force range. The second muscle activity is a muscle activity lower than the first muscle activity by a defined number, of each muscle activity obtained according to the operating conditions which are in the joint contact force range. The invention has the same advantage as the corresponding method.

BRIEF DESCRIPTION OF THE DRAWINGS

Preferred embodiments of the invention will now be described in further details. Other features and advantages of the present invention will become better understood with regard to the following detailed description and accompanying drawings where:

FIG. 1 is a block diagram showing an embodiment;
FIG. 2 is a perspective view showing a passive exercise machine in an embodiment;
FIG. 3 illustrates another example of a passive exercise machine;
FIG. 4 illustrates another example of a passive exercise machine;
FIG. 5 is a side view of an example of a motion simulator used in the embodiments;
FIG. 6 is a block diagram of the simulator used in the embodiments;
FIG. 7 illustrates model examples by an inverted pendulum in the embodiments;
FIG. 8 is an explanatory diagram of a restoring force of an inverted pendulum in the embodiments;
FIG. 9 illustrates a relationship between one link model and joint positions in the embodiments;
FIG. 10 illustrates a relationship between two link model and joint positions in the embodiments;
FIG. 11 illustrates a dynamic model of a muscle used in the embodiments;
FIG. 12 shows results obtained with respect to various operating conditions in the embodiments; and
FIG. 13 shows %MVC with respect to each of candidate conditions in the embodiments.

BEST MODE FOR CARRYING OUT THE INVENTION

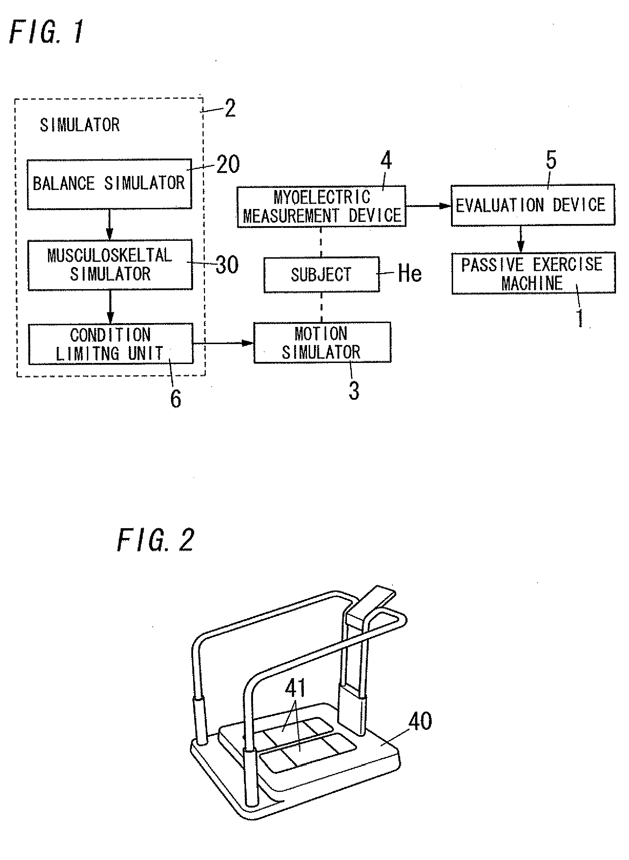

The present embodiment as a passive exercise machine includes a support base configured to support all or part of user's body weight, and moves the support base through a drive unit. For example, as shown in FIG. 2, there is a passive exercise machine 1 used by a user in the standing position. This machine 1 includes a pair of footrest bases 41 (first and second support bases) which user's right and left feet are put on, respectively, and is configured to change the positions of the footrest bases 41 through a drive unit (not shown) built in a base frame 40. The footrest bases 41 are driven by the drive unit which can drive in at least one-degree-of-freedom of a front-back direction, a right-left direction, a vertical direction, a direction around an axis in a front-back direction, a direction around an axis in a right-left direction and a direction around a vertical axis.

For example, if each footrest base 41 is moved by using a parallel link mechanism having six extensible rods of which each two rods are arranged in parallel, each footrest base 41 can be changed its own position in six-degree-of-freedom. Each footrest base 41 can be also moved in arbitrary degree-of-freedom by using a mechanism which has a motor as a drive source and is configured to perform conversion between a rectilinear movement and a turning movement trough a combination of a crank(s), a gear(s) and so on. Especially, when one drive source is used, right and left footrest bases 41 can be easily interlocked by a particular relationship.

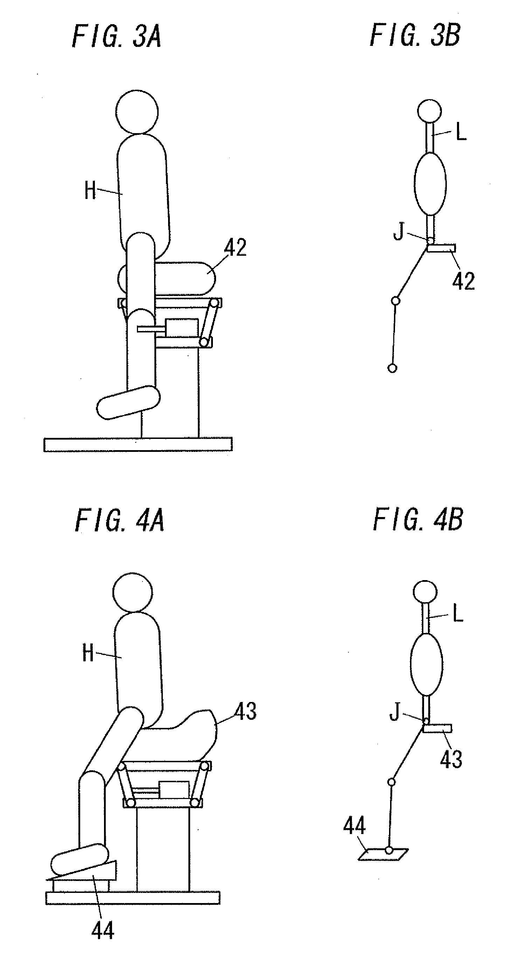

In passive exercise machines 1, as shown in FIG. 3, there is a machine which includes a support base (a seat 42) which supports the buttocks of a user H and the user H rides on. The machine provides the user H with an exercise simulating horse riding by moving the seat 42. As shown in FIG. 4, there is a machine which includes a support base (a seat 43) for supporting the buttocks or the waist of a user H and support bases (footrest bases 44) on which the right and left feet of the user H are put on, respectively. This machine is configured to change loads acting on the legs of the user H by inclining the seat 43 while supporting the user's (H) load by three-point mounting supporting to change a rate of the load supported by the seat 43. In the configuration of FIG. 4, the footrest bases 44 can move in a vertical direction in response to loads acting on the footrest bases 44, and an angle of each knee joint is kept constant.

An example applied to the passive exercise machine 1 shown in FIG. 2 is hereinafter explained, but the present invention can be also applied to a passive exercise machine 1 shown in FIG. 3 or 4.

As shown in FIG. 1, the embodiment includes a simulator 2, a motion simulator 3 and a myoelectric measurement device 4. The simulator 2 is configured to find, by a computer simulation, user's physical response when the operating condition of the support bases in the passive exercise machine 1 is changed. The motion simulator 3 is configured to simulate each motion of the support bases of the passive exercise machine 2 based on various operating conditions. The myoelectric measurement device 4 is configured to measure myoelectric potential of each target muscle with regard to a subject (He) supported by the support bases of the motion simulator 3.

An operating condition of a support base means a condition which is set by parameters such as vector, amplitude (moving range), frequency (moving velocity), etc about a moving support base. In the inner potion of the simulator 2, the operating condition is represented as time change of a representative position of a support base. However, if an operator of the simulator 2 sets the parameters (the detail will be discussed later), time change of a representative position of each support bases is automatically produced in the inner potion of the simulator 2.

Even if an operating condition is in a range that cannot be set to a real passive exercise machine 1 or is unacceptable for a user, such an operating condition can be set through the simulator 2. If different operating conditions including unrealisable operating conditions are tried through the simulator 2, it is possible to obtain a rough tendency for a relationship between an operating condition and user's physical response. Therefore, in the simulator 2, if different operating conditions are tried and applied to the support bases which a person actually gets on, it is possible to narrow down to operating conditions which an intense exercise effect can be obtained by and can be used safely.

In the configuration shown in FIG. 1, a condition limiting unit 6 is provided. In the unit 6, different operating conditions tried in the simulator 2 are narrowed down to intermediate conditions to be tried in the motion simulator 3. The simulator 2 and the condition limiting unit 6 are realized by running a program in a computer, and include a monitoring device such as a CRT or a liquid crystal display, and input devices such as a keyboard and a mouse.

Some results are obtained by trying deferent operating conditions through the simulator 2. In the condition limiting unit 6, the results are displayed on the screen of the monitoring device, and an operator can designate intermediate conditions by using the input devices. A rule may be also provided in order to decide intermediate conditions as described later (i.e., a rule for selecting operating conditions which intense exercise effect is obtained by and can provide safe use). Predetermined numbers of (e.g., about 10) intermediate conditions having higher goodness-of-fit with respect to the rule may be adopted from operating conditions complying with the rule. If such a method is adopted, intermediate conditions can be limited automatically.

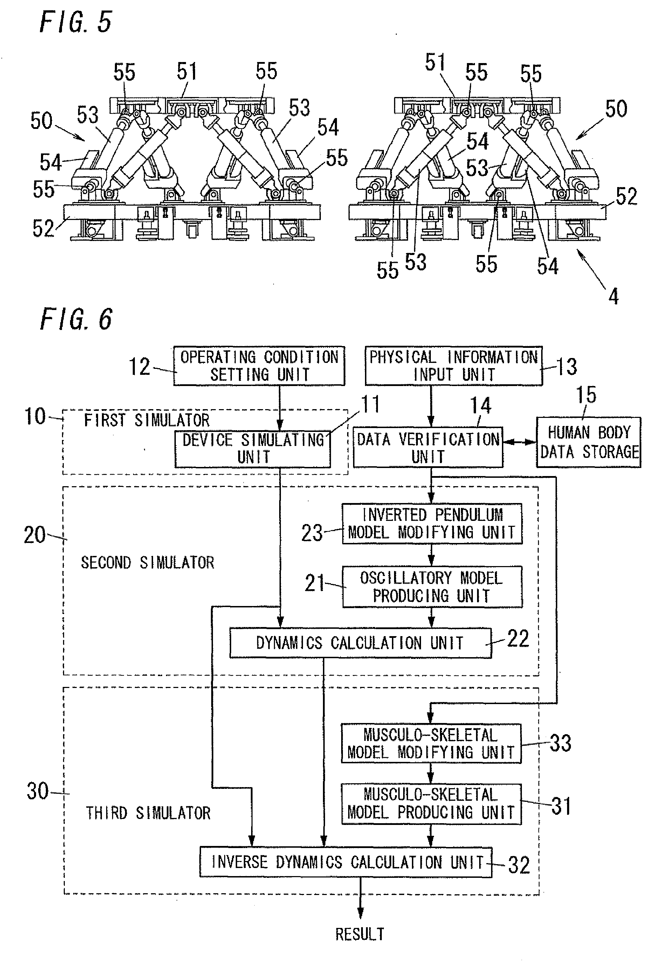

Some operating conditions adopted as the intermediate conditions though the condition limiting unit 6 are applied to the motion simulator 3. The motion simulator 3 is configured to simulate motion of each support base (footrest base 41) in the passive exercise machine 2 shown in FIG. 2. Accordingly, for example, as shown in FIG. 5 a pair of parallel link mechanisms 50 are used. The mechanisms 50 are configured to move the support bases 51 supporting the right and left feet of a subject (He) in multiple-degree-of-freedom (six-degree-of-freedom in the example shown in the figure), respectively.

A parallel link mechanism 50 is configured to support a support base 51 with six links 53 with respect to a mount base 52 and also to move the support base 51 by individually elongating and contracting links 53 through motors 54 which are placed in the vicinity of the links 53, respectively and each configured to rotate in both directions. Both ends of each of the links 53 are joined to the support base 51 and the mount base 52 through universal joints 55, respectively. In this configuration, the links 53 are elongated and contracted and thereby the support base 51 can be moved in front-back direction, a right-left direction, a vertical direction, a direction around an axis in a front-back direction, a direction around an axis in a right-left direction and a direction around a vertical axis.

A controller (not shown) is configured to control a rotating amount and rotating timing of each motor 54 of the motion simulator 3. Therefore, in order to move the support base 51 by each of the intermediate conditions adopted through the condition limiting unit 6, the controller is provided with a transformation arithmetic unit configured to transform each intermediate condition into a rotating amount and rotating timing of each motor 54. If receiving one intermediate condition, the transformation arithmetic unit carries out an operation for transforming the intermediate condition into time-series of operational amounts applied to each motor 54. That is, the transformation arithmetic unit is formed of a computer and outputs operational amounts of the motors 54 in response to an input of an intermediate condition. Therefore, the transformation arithmetic unit can be formed of the same computer as that of the simulator 2 and the condition limiting unit 6.

The motion simulator 3 is provided in order to measure a muscle activity of a subject (He) while moving the support bases 51 by an intermediate condition after the subject (He) stands on the support bases 51. Therefore, when the motion simulator 3 is driven, the myoelectric measurement device 4 detects muscle activities with regard to different muscles in target regions of the subject (He). For example, if the passive exercise machine 1 shown in FIG. 2 is used, it is considered that user's muscle activities of foot regions, lower thigh regions, femoral regions, buttocks, lower back, abdominal region and so on are enhanced. Accordingly, these muscles are set to target regions with respect to the subject (He) standing on the motion simulator 3, and each myoelectric potential is measured through the myoelectric measurement device 4.

If a plurality of (e.g., 2000) operating conditions obtained by performing a simulation through the simulator 2 are narrowed down to ten intermediate conditions, myoelectric potential measurement is performed with respect to the subject (He) in accordance with each of the ten intermediate conditions. In this instance, the myoelectric potential measurement is performed with respect to not just one subject (He) but each of subjects (He) who differ in age or gender. Preferably, such myoelectric potential measurement is performed more than one time per subject. Since the number of the intermediate conditions is about ten, actual measurement of myoelectric potential can be performed for a comparatively short time.

If myoelectric potential measurement is performed in accordance with each of the intermediate conditions, it is possible to acquire human body's muscle activities by moving the support bases 51 in accordance with each of the intermediate conditions. Accordingly, the evaluation device 5 decides an optimum intermediate condition with regard to muscle activities from the intermediate conditions, and defines the intermediate condition as an operating condition of the passive exercise machine 1. The operating condition defined by the evaluation device 5 is one condition to which the operating conditions set through the simulator 2 are finally narrowed down. Whether or not it is in a range that the passive exercise machine 1 can be used safely is verified through the simulator 2 and the motion simulator 3, and the exercise effect is verified through the motion simulator 3. Accordingly, it can be considered to be optimal as an operating condition which the passive exercise machine 1 is provided with.

The aforementioned components are explained in detail herein. As shown in FIG. 6, three kinds of simulators 10, 20 and 30 for performing a computer simulation each are combined and constitute the simulator 2. The second simulator 20 and the third simulator 30 are hereinafter referred to as a "balance simulator" and a "musculoskeletal simulator", respectively.

The first simulator 10 includes a device simulating unit 11 configured to simulate motion of each support base (foot base 41) in the passive exercise machine 1. The simulator 10 is configured to receive an operating condition provided for the passive exercise machine 1 from an operating condition setting unit 12 to simulate motion of each support base in accordance with the operating condition.

Periodicity is not indispensable to motion of each support base. It is also not required that a movement locus of each support base forms a geometric shape. However, in the embodiment, for the purpose of easy installation of an operating condition, motion of each support base has periodicity and each movement locus forms a shape represented by a reciprocating motion resultant in one or more directions. That is, each movement locus is represented by a reciprocating motion resultant arbitrarily selected from rectilinear reciprocating motion and turning reciprocating motion. The rectilinear reciprocating motion is selected from a front-back direction, a right-left direction and a vertical direction. The turning reciprocating motion is selected from a roll (a direction around an axis in a front-back direction), a pitch (a direction around an axis in a right-left direction) and a yaw (a direction around a vertical axis). (As a matter of course, in case of one-degree-of-freedom, it is presented by one reciprocating motion.)

Therefore, it is possible to set a direction of reciprocating motion deciding a movement locus of each support base, amplitude and cycle (frequency) per direction of reciprocating motion, a phase relation between each reciprocating motion when a set of reciprocating motion are add, and a reference position of reciprocating motion (the middle position of the reciprocating motion or the like), as an operating condition through the operating condition setting unit 12.

It is an object of the embodiment to estimate user's muscle activities and joint contact forces (joint forces) in response to motion of each support base of the passive exercise machine 1. Accordingly, the musculoskeletal simulator 30 uses a musculo-skeletal model having body segments (bones), joints and tissue (muscles). The joint contact force means a force acting on a joint (joint axis) in a normal direction to a contact surface between the joint (joint axis) and a body segment (link).

It is difficult to directly apply motion of each support base of the passive exercise machine to a musculoskeletal model. Therefore, in the balance simulator 20 of the embodiment, a machine vibration system's model simplified so that human motion is not essentially lost is described and used in place of a human body. The relationship between the described model and the passive exercise machine's motion simulated through the device simulating unit 11 is represented by a motion equation. The machine vibration system's model as substitution of a human body is described through the oscillatory model producing unit 21. The balance simulator 20 is also provided with a dynamics calculation unit 22 in order to carry out dynamics calculation for finding solution to the aforementioned motion equation. The dynamics calculation unit 22 finds each position change of human joints (position and velocity) when the passive exercise machine is activated.

Bending and stretching of a joint is generated by muscular contraction in relation to the joint. Accordingly, a load acting on the muscle in relation to the joint (muscle activity) can be obtained by applying a position change over time of a joint found through the balance simulator 20 to a musculo-skeletal model used by the musculoskeletal simulator 30. A load acting on a body segment is also obtained, and thereby a load acting on the joint (a joint contact force) is obtained. That is, muscle activities of muscles in relation to bending and stretching joints (loads acting on muscles) and loads acting on the joints (joint contact forces) are obtained by inverse dynamics calculation from each position change of the joints (orbits).

Therefore, the musculoskeletal simulator 30 is provided with a musculo-skeletal model producing unit 31 and an inverse dynamics calculation unit 32. The unit 31 is configured to perform definition of a musculo-skeletal model as substitution of a human body. The unit 32 is configured to calculate muscle loads and joint loads from each position change of joints found through the balance simulator 20.

The operation of the simulator 2 is explained in detail. The device simulating unit 11 focuses attention on positions of the support bases for supporting user's load and each operating condition of the support bases, but does not consider the mechanism in particular. In other words, the device simulating unit 11 simulates which region(s) of a human body is supported by the support bases, and how and where of a contact region(s) is moved. A region(s), supported by the support bases, of a human body is a contact region(s) which is in contact with the support bases, and the contact region(s) is a restraint condition that restrains the human body's movement. Degree-of freedom on each motion of the support bases is included in a direction of a reciprocating motion in an operating condition.

The passive exercise machine 1 in the embodiment supports both feet of a user and moves each foot in six-degree-of-freedom (six directions). Accordingly, the device simulating unit 11 requires an operating condition of six-degree-of-freedom defining each movement of the footrest bases 41. However, the describing amount of the operating condition can be reduced by using a constrained condition that the right and left footrest bases 41 symmetrically moves in the same phase or in reverse phase, as compared with individually defining operating conditions with respect to footrest bases 41.

In the inner portion of the simulator 2, an operating condition is represented by time change of a representative position of each support base 41, as discussed previously. However, an operating condition entered through the operating condition setting unit 12 by an operator is represented by parameters such as motion trajectory, frequency, amplitude, phase, etc.

Herein, it is assumed that the footrest bases 41 are moved along the upper surface of the base frame 40 put on the floor face or the like. In the actual passive exercise machine 1, each footrest base 41 can provide turning reciprocating motion around a pivot along the upper surface of the base frame 41 and turn foot rested on the footrest base 41 around the ankle joint (at least one of plantarflexion and dorsiflexion). However, for the purpose of a simple simulation, the balance simulator 20 in the embodiment does not include a simulation of the operation.

A motion trajectory is a movement locus of a representative position of a support base 41 on a plane along the upper surface of the base frame 40, and can be selected from movement loci such as a straight line, an arc shape, an eight shape, etc. A frequency is the number of reciprocating motion of a support base 41 per second and defines a motion velocity of a support base 41. Amplitude defines a movement distance when a support base 41 reciprocates. A phase can be selected from an operation when the right and left support bases 41 move to the same side and an operation when the right and left support bases 41 move to the opposite sides. The former is an operation that, when one of the support bases 41 is at the front end position of its moving range, the other is placed at the front end position in its moving range, and is referred to as "same phase". The latter is an operation that, when one of the support bases 41 is at the front end position of its moving range, the other is placed at the rear end position in its moving range, and is referred to as "reverse phase".

In addition to the above ones, there are, as parameters, an angle of a moving direction of a support base 41 with respect to a front-back direction of the base frame 40 (an inclined angle), an average distance between representative points (e.g., center points) of the right and left support bases 41 (feet width) and the like. In the embodiment, plantarflexion and dorsiflexion are excluded in the balance simulator 20, but when plantarflexion and dorsiflexion are performed by turning a support base 41 up and down, the parameters can include a position, an orientation, a turning angle range (turning amplitude) about a pivot of a footrest base 41 as well as an angle of the center position of the turning angle range with respect to the upper surface of the base frame 40 (an offset angle).

In the embodiment, a left-handed orthogonal coordinate system is used for a coordinate system for representing a moving direction, and turning about each coordinate axis is right-hand turning (clockwise) towards a positive direction of each coordinate axis. A rectilinear motion towards a direction of each coordinate axis is provided with an arbitrary position as a reference position of which phase is 0°. A turning motion around each coordinate axis is provided with a direction of one coordinate axis included in a plane perpendicular to the coordinate axis itself, as a reference position of which phase is 0°. For example, when an XYZ orthogonal coordinate system is used, a reference position of turning motion around the X-axis is a direction of Y-axis or Z-axis included in a YX plane perpendicular to the X-axis, which is a reference position of which phase is 0°.

In the passive exercise machine 1 of the embodiment, each footrest base 41 is provided with a coordinate system. A foot size is disregarded and an ankle joint is regarded as a joint axis around a Y-axis. The position of a footrest base's (41) upper surface which is horizontal is set as a reference position around an X-axis and a Y-axis. An orientation from a heel to a toe is defined as the positive direction of an X-axis.

When receiving the aforementioned parameters, the device simulating unit 11 calculates position change over (accompanying) time of a footrest base 41. That is, a time series of positions with regard to a footrest base 41 is output at regular time intervals.

Conditions about individual body type are required in order to describe a model (human body model) as substitution of a human body in the balance simulator 20 and the musculoskeletal simulator 30. Describing the model is important to evaluate a muscle load and a joint load, and the conditions about individual body type require not only body height and body weight but also age and gender for deciding muscle distribution. Conditions of body fat amount, muscle mass and so on are also useful for enhancing description accuracy of the model. For example, "body height: 170cm, body weight: 60kg, age: 70, gender: male" are provided for the balance simulator 20 and the musculoskeletal simulator 30 as the conditions about individual body type. The conditions of body type are entered through the physical information input unit 13. In the embodiment, (body height, body weight, age, gender) are entered through the physical information input unit 13.

Body type information entered through the physical information input unit 13 is checked with the human body data storage 15 which relates a combination of four - (body height, body weight, age, gender) to (body segment length, body segment mass, body segment's inertia moment) to register them. A data set of the human body data storage 15 is obtained and used from statistical values of a human body. However, in order to reduce a calculation amount, body segment length (upper body length, thigh length, etc.) and body segment mass are related to (body height, body weight) without consideration of age, gender and body segment's inertia moment.

Statistical values based on anatomy can be used for body segment length and mass with respect to (body height, body weight). However, body segment length and mass with respect to standard values of (body height, body weight) may be provided. In this instance, when (body height, body weight) are entered through the physical information input unit 13, body segment length and mass are calculated by proportional calculation with respect to the standard values (or another suitable relational expression). In case of the calculation, age and gender are considered. Inertia and restoration elements need to be considered as elements of a human body model as referred to hereinafter. Accordingly, it is desirable that strength of a restoring force is also stored in the human body data storage 15. Standard values may be provided for the restoring force and corrected by using data on a muscle force per age and gender.

In the embodiment, a machine vibration system's model is described as substitution of a human body in the balance simulator 20, as discussed previously. That is, a foot rested on a footrest base 41 is a contact region and there is a restraint condition that the foot position is restrained by the support base. When the footrest base 41 is periodically moved, a user tries to stand erect while keeping maintain user's balance.

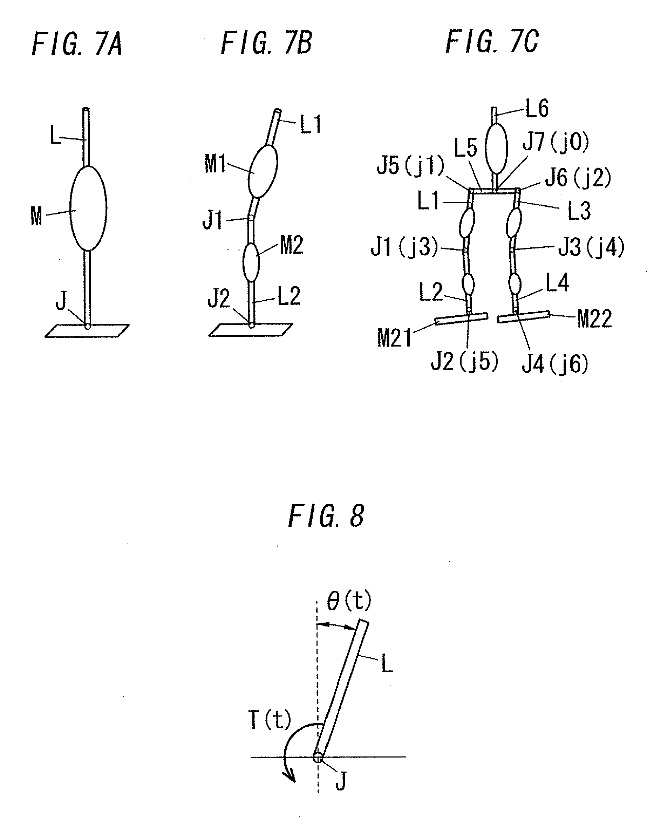

Therefore, as shown in FIG. 7A, an inverted pendulum formed of one link (L) can be used as the most simplified machine vibration system's model. The inverted pendulum has mass (M) equivalent to user's body weight and is hereinafter referred to as "one link model". In order to analyze action of the inverted pendulum, the pendulum is provided with three elements - an inertia element (an element exerting a force proportional to acceleration: including gravity), an attenuation element (an element exerting a force proportional to velocity) and a restoration element (an element exerting a force proportional to displacement).

In the passive exercise machine 1, moving a footrest base 41 periodically is represented by motion for periodically changing the lower end position of the link (L). In the one link model, the link (L) can be turned only about the joint axis (J) placed at the lower end position. That is, the inertia, attenuation and restoration elements of the link (L) act on around the joint axis (J). Therefore, the motion of the inverted pendulum can be described by a motion equation including the inertia, attenuation and restoration elements. On the other hand, sine wave oscillation of each direction is used as a force acted on the inverted pendulum by the passive exercise machine 1 as discussed previously. Accordingly, the dynamics calculation unit 22 can calculate time change of the position of the inverted pendulum by resolving a motion equation of forced oscillation.

The model shown in FIG. 8 is used to find the torque T(t) acting on around the joint axis (J) at time t. When the body segment (link L) is turned by the angle θ (t) with respect to the reference angle $\theta_0$ around the joint axis (J), the torque T(t) can be represented by T(t)=Kp · (θ(t) · $\theta_0$) + Kd · dθ(t)/dt.

The angle θ(t) is a joint angle and equivalent to an inclined angle with respect to an erect position of the link (L) in the one link model. Around the joint axis (J) between a pair of links, the angle becomes an angle between the links. An absolute value of angle between the links may be used for the joint angle (J) or an angle measured by defining a joint angle at rest (resting standing position in case of standing position) as a reference angle may be used.

In this regard, Kp is a proportional gain and Kd is a differential gain. The proportional gain (Kp) and the differential gain (Kd) are obtained from a muscle force of a human body as a model. The torque T(t) may be only a value proportionate to the joint angle θ(t), or an integral value of the joint angle θ(t) may be added, or a time delay may be added in consideration of a response time of a human body. Like general PID control, a term of the formula 1 may be also added. In the formula 1, Ki is an integral gain.

[Formula 1]

$$Ki \int_0^t \{\theta(t)\} d\theta$$

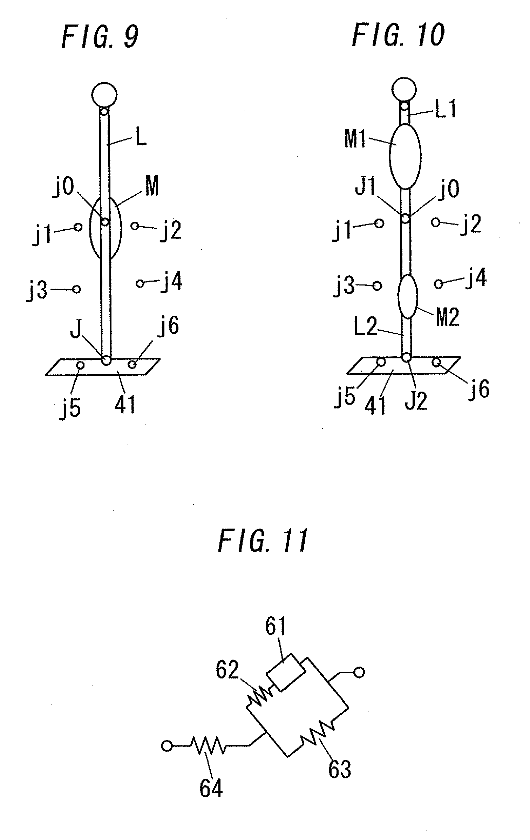

In the above-mentioned inverted pendulum model, each position of joints is unknown. Accordingly, it is impossible to apply joint positions to a musculo-skeletal model used by the musculoskeletal simulator 30. Therefore, as shown in FIG. 9, in the default position of the actual passive exercise machine 1, a position relation between a human body's reference point (e.g., gravity) and each joint is actually measured. The position relation is then applied to the position of a reference point (e.g., gravity) of the link (L). Thereby, each position of the joints (j0-j6) is estimated from the position of the reference point of the inverted pendulum. In the example shown in the figure, j0 represents a waist joint, j1 and j2 represent hip joints, j3 and j4 represents knee joints, and j5 and j6 represent ankle joints. When the position relation between the human body's reference point and each joint is actually measured, a device such as motion capture or the like is used.

Since one link model is simple, the calculation amount is small. However, each position of the joints (j0-j6) is estimated from a position of one reference point, and accordingly the estimated positions of the joints (j0-j6) cannot be regarded as being high accuracy. Therefore, as shown in FIG. 7B, a human body may be represented by a model formed of two links (L1 and L2) having mass (M1) and mass (M2) of an upper body and a lower body, respectively as well as two joint axes (J1 and J2) (hereinafter referred to as "two link model").

In this model, as shown in FIG. 10, one reference point (e.g., waist joint or the like) is related to one joint axis (J1), and a reference point (e.g., gravity) is set to each of the links (L1 and L2). In this regard, in the embodiment, each motion of the joints (j0-j6) in the lower body is considered. Accordingly, the link (L1) corresponding to the upper body of the links (L1 and L2) is used as only a factor influencing the movement of the link (L2) corresponding to the lower body. The position relation between each of the joints (j0-j6) and the reference point of the link (L) is unnecessary.

It is considered that a model including the same number of body segments and joint axes (joints j0-j6) as a musculo-skeletal model as shown in FIG. 7C (hereinafter referred to as "multi-link model") is used in order to precisely detect each joint position in an inverted pendulum model. In the multi-link model, it is possible to obtain each individual position of the joints (j0-j6). Accordingly, each position of the joints (j0-j6) obtained through the balance simulator 20 can be applied to the musculoskeletal simulator 30 without change. However, since the balance simulator 20 calculates each individual position of the joints (j0-j6), the calculation amount is increased substantially.

In FIG. 7C, M21 and M22 are models of first and second support bases, respectively. For example, the multi-link model (a human body model) includes first to sixth links (L1-L6). The first link (L1) has a first joint axis (J1) that is equivalent to the joint (j3) and is placed at the lower end of the first link (L1). The second link (L2) has a second joint axis (J2) that is equivalent to the joint (j5) and is placed at the lower end of the second link (L2). The upper end of the second link (L2) is joined to the lower end of the first link (L1) through the first joint axis (J1), while the lower end of the second link (L2) is joined to the model (M21) of the first support base through the second joint axis (J2). The third link (L3) has a third joint axis (J3) that is equivalent to the joint (j4) and is placed at the lower end of the third link (L3). The fourth link (L4) has a fourth joint axis (J4) that is equivalent to the joint (j6) and is placed at the lower end of the fourth link (L4). The upper end of the fourth link (L4) is joined to the lower end of the third link (L3) through the third joint axis (J3), while the lower end of the fourth link (L4) is joined to the model (M22) of the second support base through the fourth joint axis (J4). The fifth link (L5) has fifth and sixth joint axes (J5 and J6) that are equivalent to joints (j1 and j2), respectively and are placed at both ends of the fifth link (L5). Both ends of the fifth link (L5) are also joined to the upper ends of the first and third links (L1 and L3) through the fifth and sixth joint axes (J5 and J6), respectively. The sixth link (L6) has a seventh joint axis (J7) that is equivalent to the joint (j0) and is placed at the lower end of the sixth link (L6). The lower end of the sixth link (L6) is also joined to the center of the fifth link (L5) through the seventh joint axis (J7). A restoring force responding to an angle between the first and second links (L1 and L2) acts on around the first joint axis (J1). A restoring force responding to an angle between the third and fourth links (L3 and L4) acts on around the third joint axis (J3). A restoring force responding to an angle with respect to an erect position of the second link (L2) acts on around the second joint axis (J2). A restoring force responding to an angle with respect to an erect position of the fourth link (L4) acts on around the fourth joint axis (J4).

In the balance simulator 20, the calculation amount and accuracy are in trade-off. Accordingly, it is desirable to select a model as the need arises. In an example of use, for schematic evaluation, a range of operating conditions is narrowed down by using the one link model of FIG. 7A or the two link model of FIG. 7B. The evaluation can be subsequently performed by using the multi-link model of FIG. 7C within the narrowed range.

Data extracted from the human body data storage 15 are each body segment's length and mass. Accordingly, when one link model or two link model is used, link length, link mass and restoring force need to be modified and the data extracted from the human body data storage 15 cannot be used without change. Therefore, in the inverted pendulum model modifying unit 23, the data extracted from the human body data storage 15 are modified in response to a used model, and thereby link length, link mass and a restoring force are obtained. In each example of FIGS. 9 and 10, a model that a head region is separated is shown but in the embodiment, calculation is performed without separating the head region.

Each position of the joints (j0-j6) is obtained at regular time intervals. That is, the dynamics calculation unit 22 calculates position change over time of each of the joints (j0-j6). In other words, a time series of positions each of the joints (j0-j6) is output.

The musculoskeletal simulator 30 applies, to a musculo-skeletal model, time change of positions each of the joints (j0-j6) obtained through the dynamics calculation unit 22 of the balance simulator 20, and calculates each muscle load and each joint load by inverse dynamics calculation. The musculo-skeletal model is set by using (body height, body weight, age, gender) entered through the physical information input unit 13 like a human body model of an inverted pendulum. That is, the data verification unit 14 checks the data entered through the physical information input unit 13 with the human body data storage 15 and thereby uses obtained body segment length, body segment mass, body segment's inertia moment and so on. The data extracted from the human body data storage 15 are modified through the musculo-skeletal model modifying unit 33 as the need arises. The musculo-skeletal model producing unit 31 produces a musculo-skeletal model by using the modified data as the need arises.

In the musculo-skeletal model, a line that doe not elongate and contract muscle as well as a bone are presented as a link of a rigid body, and a joint is represented as a joint axis. The inverse dynamics calculation unit 32 in the musculoskeletal simulator 30 applies each position change of the joints (j0-j6) obtained by using an inverted pendulum in the balance simulator 20 as position change of a joint axis of the musculo-skeletal model. In the musculo-skeletal model, each link is provided with a muscle according to a dynamic model of a muscle, and a load acting on a muscle or a joint is calculated by inverse dynamics calculation.

A Hill model is used as a dynamic model of a muscle. That is, as shown in FIG. 11, a contractile component (in which a tension generator and an attenuation element are joined in parallel) and an elastic element 62 are joined in series, and an elastic element 63 are further joined in parallel, which simulate a muscle function. In addition, in the example shown in the figure, an elastic element 64 is joined in series with the muscle, thereby simulating a tendon function.

A muscle model applied to a musculo-skeletal model is not limited to the configuration shown in FIG. 11. That is, it may be a simple configuration simulating only by an elastic element in order to reduce the calculation amount, or a configuration that a contractile speed is considered in order to enhance the accuracy.

The inverse dynamics calculation unit 32 calculates a muscle load or a joint load by performing an inverse dynamics calculation based on time change of each region position of a musculo-skeletal model. Accordingly, the unit 32 requires position change of a footrest base 41 produced through the device simulating unit 11 and each position change of the joints (j0-j6) obtained through the dynamics calculation unit 22 besides a musculo-skeletal model produced through the museum-skeletal model producing unit 31. A time series of these positions is applied to the musculo-skeletal model, and thereby a time series of muscle activities or joint contact forces can be calculated.

There are a plurality of muscles participating in a series of movements, and accordingly solutions are obtained in general by calculating a muscle or joint load from each position change of joints. Therefore, it is necessary to narrow down the solutions by some sort of prescript (rule). In the embodiment, the solutions are narrowed down based on a rule that a living thing selects a solution having better efficiency of low energetic consumption. That is, a solution having a minimal sum of muscle usage (e.g., a ratio, expressed in percentage, of an actual available muscle force to an available maximum muscle force) is selected from the solutions. Not only muscle usage but also a value obtained by multiplying muscle volume and muscle usage or a rate of slow muscle and fast muscle usage may be used for an indicator for selecting a solution.

In case a passive exercise machine 1 simulated with the device simulating unit 11 is used, it is possible to calculate each muscle load and each joint load by different operating conditions or body types, by repeating a process of the balance simulator 20 and the musculoskeletal simulator 30 after changing an operating condition set through the operating condition setting unit 12 or a body type condition entered through the physical information input unit 13.

Evaluation of calculation results differs in response to an intended purpose of a passive exercise machine 1, but it is considered that calculation results are often evaluated so that a condition for stimulating a muscle activity of a target muscle and reducing a joint contact force of each joint is fulfilled. A target muscle differs in response to a purpose of operation and exercise of a passive exercise machine 1. For example, in order to protect aged person from a fall, the person's muscle groups may be strengthened, or muscle groups around knees or lower back may be strengthened for prevention of pain in knee or backache.

A muscle activity is evaluated by using an average (value) of a set of muscle activities in a target muscle (an average during a period of a footrest base 41), an average of a set of muscle activities in all muscles configured with a musculo-skeletal model, or the like. A target muscle is set by specifying each muscle (e.g., a medial great muscle and a lateral great muscle), specifying as a muscle group of a specific region of a body (a femoral region, a leg region, etc.) or specifying as a muscle group by function (spreading muscle group of knee joint, plantarflexion muscle group of ankle joint, etc.). In the passive exercise machine 1 of FIG. 2, target muscles are, for example, femur stretching (musculus rectus femoris, lateral great muscle, medial great muscle), femur bending (long head of biceps femoris muscle), lower thigh dorsiflexion (anterior tibial muscle), lower thigh bending (gastrocnemial muscle, musculus soleus).

A joint contact force is evaluated by using a force acting on a specific joint (knee joint, ankle joint, articulatio coxae, etc.), an angle of the specific joint or the like. For example, a force in a shear direction acting on a knee joint is evaluated by a constrained condition equal to or less than 400N, and an angle of a knee joint is evaluated by a constrained condition equal to or less than 100° (a position when the knee joint is stretched is defined as 0°). It is desirable that these constrained conditions are previously provided as limit values and a decision whether outside the scope of the limit values or not is automatically made in response to an operating condition or a body type.

It is possible to obtain a safely usable operating condition for providing a large muscle activity and a small joint contact force with respect to a target muscle(s), by evaluating a load acting on each of muscles and joints calculated through the musculoskeletal simulator 30 as discussed previously.

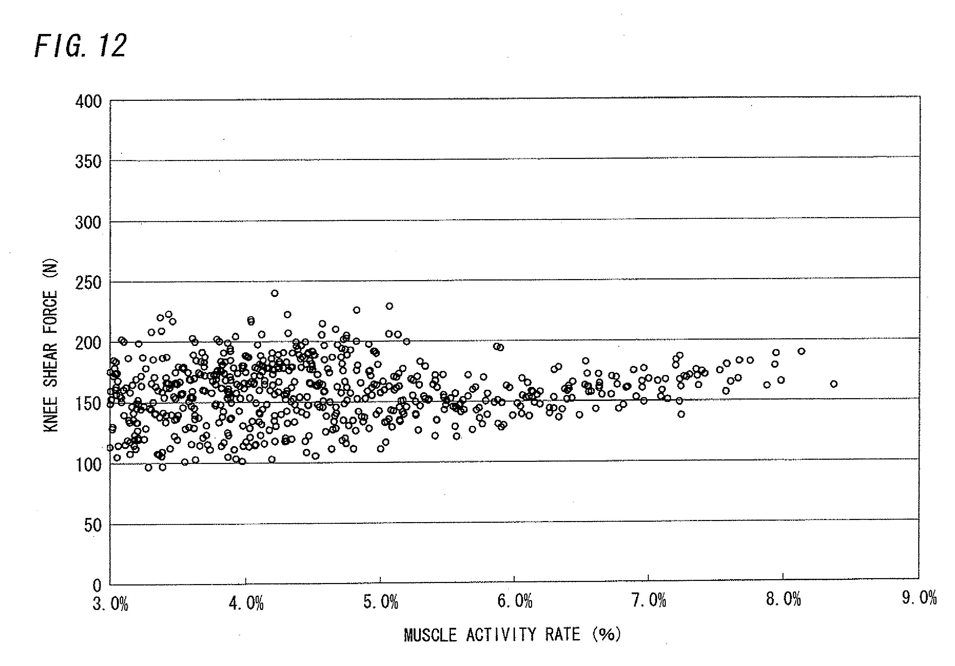

FIG. 12 shows simulation results about approximately 2000 kinds of operating conditions by, for example, obtaining a joint contact force of each knee joint, i.e., a knee shear force as well as an average muscle activity rate about all target muscles per operating condition, where a horizontal axis represents a muscle activity rate and a vertical axis represents a knee shear force. In case of a simulation, it is desirable to be exclude apparently invalid operating conditions in an actual passive exercise machine 1 and operating conditions of which each muscle activity is known to be considerable low with respect to an objective level. However, preferably operating conditions are included even if expected to be out of a range.

From the obtained results as shown in FIG. 12, the upper limit of a range that a user does not feel pain in the user's knee(s) can be set with regard to a shear force (joint contact force). Accordingly, in the condition limiting unit 6, the upper limit is defined with regard to the shear force, and an intermediate condition(s) is extracted in a range (joint contact force range) that a knee shear force is smaller than the upper limit. That is, a target range of joint contact forces is set to equal to or less than a predetermined specified value, and a selection range of intermediate conditions is narrowed down to a safely usable range.

In the embodiment, a rule set for extracting an intermediate condition includes:

(1) a rule that a knee shear force is smaller than the upper limit; and
(2) a rule that a larger muscle activity rate is in order (muscle activity range). That is, an intermediate condition is an operating condition that meets the rule (1) and the rule (2).

However, in a simulation, user's motion based on the prediction when an actual machine is used, and individual difference between users cannot be considered. Accordingly, the effect when the actual machine is used needs to be checked through an effect by a subject. Therefore, a plurality of intermediate conditions are narrowed down from simulation result. In the embodiment, ten intermediate conditions are extracted based on simulation result by the simulator 2.

In the example of FIG. 12, the upper limit of knee shear force is set to 400 [N] in order to apply the aforementioned rule (1). Top ten of muscle activity rates are extracted, so as to meet the rule (2), within a range that a knee shear force is smaller than 400 [N] (a range surrounded with a dashed ellipse). Operating conditions corresponding to the extracted results are dealt as intermediate conditions. The computer includes the simulator 2 and the condition limiting unit 6. Accordingly, all operating conditions to be simulated through the simulator 2 are stored in a storage device (not shown). The ten corresponding operating conditions selected from the simulation result are read from the storage device and then dealt as intermediate conditions. The intermediate conditions are stored in a different region from the operating conditions in the storage device.

A muscle activity and a joint contact force differ in not only operating condition but also body type. Therefore, it is possible to obtain a body type range that adequate exercise can be performed with respect to a particular operating condition, or to obtain an operating condition that adequate exercise can be performed with respect to a particular body type. It is desirable that these results are stored in the storage device and thereby can be referred all of the time.

The intermediate conditions adopted through the condition limiting unit 6 based on simulation results by the simulator 2 are applied to the aforementioned motion simulator 3. Myoelectric potential is then measured through the myoelectric measurement device 4 with respect to a subject getting on the support bases 51 of the motion simulator 3. The influence of individual difference can be easily removed by more subjects. In addition, it is desirable that body types, ages and genders are dispersed.

After the myoelectric measurement device 4 obtains myoelectric potential with regard to each muscle, the evaluation device 5 extracts an operating condition that is a maximum average value or a maximum peak value for amount sets of muscle discharge (electrical activity), from measured myoelectric potential. The average value for amount sets of muscle discharge means a time integration value of amount of muscle discharge obtained from every muscle. An average value or a peak value for amount sets of muscle discharge are found per subject and per muscle. Accordingly, for easy comparison of intermediate conditions, the evaluation device 5 averages, over subjects, a set of average values or peak values of amount of muscle discharge, and further calculates a summation per muscle. The obtained value represents muscle activity degree per intermediate condition. Accordingly, in the evaluation device 5, an intermediate condition that a muscle activity becomes maximum is selected as an operating conditions installed on an actual machine.

In the above example, an absolute value of amount of muscle discharge per muscle is used. However, in order to evaluate more exactly, it is desirable that an amount of muscle discharge is normalized. That is, preferably it is normalized by obtaining a ratio (%MVC) of a muscle contraction force (an average value or a peak value) to maximum voluntary contraction (MVC) per muscle. An intermediate condition is then evaluated by using a value obtained by averaging, over subjects, a sum (or an average), relating to all muscles, of a set of %MVC obtained with regard to each muscle.

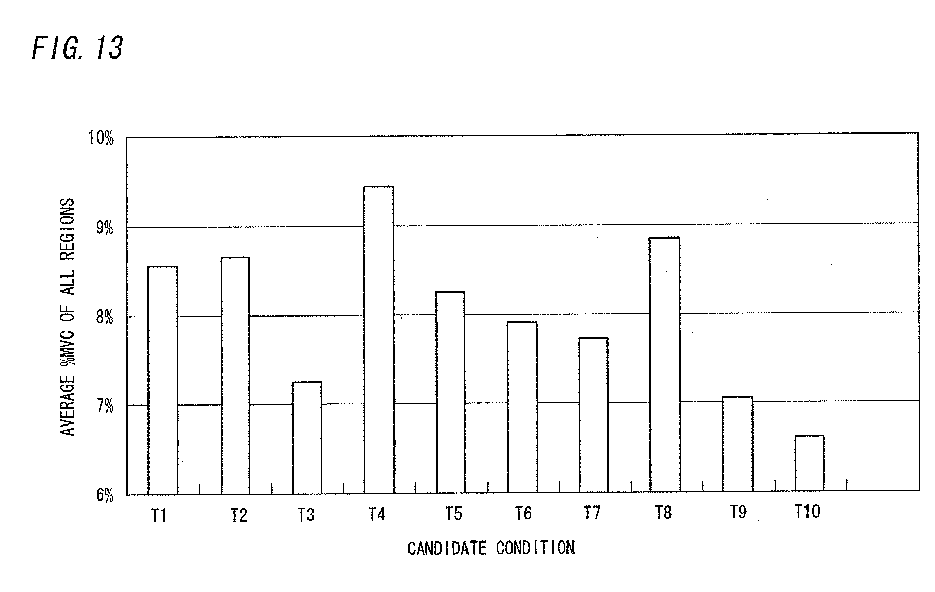

FIG. 13 shows an example - values each of which is obtained by averaging, over subjects, a set of averages each of which is an average of a set of %MVC (an average %MVC) obtained relating to all target muscles with regard to ten kinds of intermediate conditions. In this example, since a muscle contraction force of each muscle is normalized by maximum voluntary contraction, it is possible to compare a muscle activity with the same reference irrespective of type of muscle. FIG. 13 shows amplitudes of muscle activities with regard to intermediate conditions (T1-T10) (an average %MVC over muscles in all regions). The average %MVC obtained by the intermediate condition (T4) is maximum among those of the ten intermediate conditions. Accordingly, the intermediate condition (T4) is selected as an operating condition installed to an actual machine. Thus, by using an indicator for a muscle activity such as %MVC, and the evaluation device 5 selects the condition providing the maximum %MVC among the intermediate conditions as an operating condition installed to an actual machine. Accordingly, it is possible to automatically select an operating condition installed to an actual passive exercise machine 1 from intermediate conditions through the evaluation device 5.

In the aforementioned example, myoelectric potential is measured through the myoelectric measurement device 4 in order to quantify a muscle activity. However, any other value may be used if being a quantitatively measurable index of a muscle activity, such as measurement of oxygen consumption by a near-infrared spectroscopic method or the like.

In the aforementioned example, the passive exercise machine 1 is used with a user being in a standing position as shown in FIG. 2, and a restraint condition of foot placement is used in the balance simulator 20. However, in the passive exercise machine 1 of FIG. 3, hip joint positions are restrained by the seat 42. That is, the hip joint positions are determined by an operating condition of a seat 42 (a support base). Therefore, it is possible to use, as an inverted pendulum, one link model including a link (L) equivalent to an upper body as shown in FIG. 3B, besides a multiple link model. In the example shown in the figure, the lower end is a joint axis (J).

In the passive exercise machine 1 shown in FIG. 4, positions of hip joints are restrained by the seat 43, and positions of ankle joints are restrained by footrest bases 44. In other words, the positions of the hip joints and the ankle joints are determined by an operating condition of the seat 43 (a support base). In this passive exercise machine 1, it is possible to use one link model including a link (L) equivalent to an upper body in FIG. 4B besides a multiple link model in the same way as the passive exercise machine 1 shown in FIG. 3. In the example shown in the figure, the lower end is a joint axis (J).

In any case of the passive exercise machine 1 shown in FIG. 3 or the passive exercise machine 1 shown in FIG. 4, when one link model is used, a target joint can be estimated by a position relation with respect to a reference point like the case that one link model shown in FIG. 7A is used.

In seating state on a seat (42 or 43) of a passive exercise machine 1 shown in FIG. 3 or 4, if contact regions between a human body and a seat (42 or 43) need to be divided into points, a result obtained by finding position change per contact region from an operating condition may be reflected in motion of a model. In addition, although an upper body is simulated by one link mode, an inverted pendulum having links in which suitable joint axes are placed at backbone and neck positions may be used as a model.

Although the present invention has been described with reference to certain preferred embodiments, numerous modifications and variations can be made by those skilled in the art without departing from the true spirit and scope of this invention.

**Claims**

1. A method of simulation and measurement related to an optimum operating condition for a support base of a passive exercise machine,
the passive exercise machine comprising the support base configured to support all or part of body weight of a user, and a drive unit configured to move the support base, the passive exercise machine being configured to provide passive exercise for the user by moving the support base through the drive unit in accordance with an operating condition,
wherein the method comprises computer implemented steps of:

(a) obtaining different muscle activities and different joint contact forces in a target region of the user by a computer simulation sequentially according to each of operating conditions, the different muscle activities being activities of different muscles, the different joint contact forces being joint contact forces on different joints;
(b) obtaining intermediate conditions from the operating conditions, the intermediate conditions being obtained by, if every muscle activity and every joint contact force obtained sequentially according to each of the operating conditions are in predetermined muscle activity and joint contact force ranges, respectively, including the operating condition among the intermediate conditions;
(c) measuring myoelectric potential of a subject on the support base while controlling a motion simulator configured to move the support base in degrees-of-freedom sequentially according to each of the intermediate conditions; and
(d) deciding and outputting an optimum operating condition from the intermediate conditions based on each myoelectric potential measured sequentially according to each of the intermediate conditions.

2. The method of claim 1, wherein the step (a) comprising:

estimating position change over time of a human body joint of the user from position change over time of at least one inverted pendulum when the inverted pendulum is forcibly oscillated sequentially according to each of the operating conditions, the inverted pendulum being a human body model; and
obtaining the different muscle activities and the different joint contact forces by applying the estimated position change to a musculo-skeletal model.

3. The method of claim 1, wherein the step (d) comprises:

obtaining a maximum average value or a maximum peak value of muscle discharge from each myoelectric potential measured sequentially according to each of the intermediate conditions; and
defining the intermediate condition corresponding to the maximum average value or the maximum peak value as the optimum operating condition.

4. The method of claim 1,
wherein the joint contact force range is equal to or less than a specified value, and
wherein the muscle activity range is a range from a first muscle activity to a second muscle activity, the first muscle activity being the largest muscle activity of each muscle activity obtained according to the operating conditions which are in the joint contact force range, the second muscle activity being a muscle activity lower than the first muscle activity by a defined number, of each muscle activity obtained according to the operating conditions which are in the joint contact force range.

5. A system of simulation and measurement related to an optimum operating condition for a support base of a passive exercise machine,
the passive exercise machine comprising the support base configured to support all or part of body weight of a user and a drive unit configured to move the support base, the passive exercise machine being configured to provide passive exercise for the user by moving the support base through the drive unit in accordance with an operating condition,
wherein the system comprises:

a simulator configured (i) to obtain different muscle activities and different joint contact forces in a target region of the user by a computer simulation sequentially according to each of operating conditions, and (ii) to obtain intermediate conditions from the operating conditions, the different muscle activities being activities of different muscles, the different joint contact forces being joint contact forces on different joints, the intermediate conditions being obtained by, if every muscle activity and every joint contact force obtained sequentially according to each of the operating conditions are in predetermined muscle activity and joint contact force ranges, respectively, including the operating condition among the intermediate conditions;

a motion simulator configured to move the support base in degrees-of-freedom sequentially according to each of the intermediate conditions;

a myoelectric measurement device configured to measure myoelectric potential of a subject on the support base; and

an evaluation device configured to decide and output an optimum operating condition from the intermediate conditions based on each myoelectric potential measured sequentially according to each of the intermediate conditions.

6. The system of claim 5, wherein the simulator comprising:

a balance simulator configured to estimate position change over time of a human body joint of the user from position change over time of at least one inverted pendulum when the inverted pendulum is forcibly oscillated sequentially according to each of the operating conditions, the inverted pendulum being a human body model; and

a musculoskeletal simulator configured to obtain the different muscle activities and the different joint contact forces by applying the estimated position change to a musculoskeletal model.

7. The system of claim 5, wherein the evaluation device is configured: to obtain a maximum average value or a maximum peak value of muscle discharge from each myoelectric potential measured sequentially according to each of the intermediate conditions; and to define the intermediate condition corresponding to the maximum average value or the maximum peak value as the optimum operating condition.

8. The system of claim 5,

wherein the joint contact force range is equal to or less than a specified value, and

wherein the muscle activity range is a range from a first muscle activity to a second muscle activity, the first muscle activity being the largest muscle activity of each muscle activity obtained according to the operating conditions which are in the joint contact force range, the second muscle activity being a muscle activity lower than the first muscle activity by a defined number, of each muscle activity obtained according to the operating conditions which are in the joint contact force range.

## FIG. 1

## FIG. 2

## FIG. 3A

H

42

## FIG. 3B

L

J

42

## FIG. 4A

H

43

44

## FIG. 4B

L

J

43

44

## FIG. 5

## FIG. 6

RESULT

## FIG. 7A

L

M

J

## FIG. 7B

L1

M1

J1

M2

J2   L2

## FIG. 7C

L6

L5   J7 (j0)

J5 (j1)

L1   J6 (j2)

L3

J1 (j3)   J3 (j4)

L2   L4

M21   M22

J2 (j5)   J4 (j6)

## FIG. 8

$\theta$(t)

L

T(t)

J

FIG. 9

FIG. 10

FIG. 11

## FIG. 12

EP 2 305 355 A1

*FIG. 13*

CANDIDATE CONDITION

## INTERNATIONAL SEARCH REPORT

| International application No. |
| --- |
| PCT/JP2009/061389 |

**A. CLASSIFICATION OF SUBJECT MATTER**
*A63B23/00*(2006.01)i

According to International Patent Classification (IPC) or to both national classification and IPC

**B. FIELDS SEARCHED**

Minimum documentation searched (classification system followed by classification symbols)
A63B23/00

Documentation searched other than minimum documentation to the extent that such documents are included in the fields searched
| | | | |
| --- | --- | --- | --- |
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2006-34640 A (Matsushita Electric Works, Ltd.), 09 February, 2006 (09.02.06), Par. Nos. [0030] to [0047]; all drawings & WO 2006/011408 A1  & US 2008/0312040 A1 & EP 1779904 A1  & KR 10-2007-0039965 A & CN 101022855 A  & TW 281856 B | 1-8 |
| Y | JP 2005-527004 A (Honda Motor Co., Ltd.), 08 September, 2005 (08.09.05), Full text; all drawings & WO 2003/002967 A2  & US 2003/0018283 A1 & EP 1399063 A | 1-8 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 13 July, 2009 (13.07.09) | 21 July, 2009 (21.07.09) |

| Name and mailing address of the ISA/ | Authorized officer |
| --- | --- |
| Japanese Patent Office | |
| Facsimile No. | Telephone No. |

Form PCT/ISA/210 (second sheet) (April 2007)

**INTERNATIONAL SEARCH REPORT**

| International application No. |
| --- |
| PCT/JP2009/061389 |

C (Continuation).    DOCUMENTS CONSIDERED TO BE RELEVANT

| Category* | Citation of document, with indication, where appropriate, of the relevant passages | Relevant to claim No. |
| --- | --- | --- |
| Y | JP 2007-334446 A  (Matsushita Electric Works, Ltd.), 27 December, 2007 (27.12.07), Full text; all drawings (Family: none) | 1-8 |
| Y | JP 10-230004 A  (Yaskawa Electric Corp.), 02 September, 1998 (02.09.98), Full text; all drawings (Family: none) | 1-8 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004344684 A
- JP 2006122595 A
- JP 2006034640 A